# EUROPÄISCHE PATENTSCHRIFT

(11) **EP 4 574 180 B1**
(45) Veröffentlichungstag und Bekanntmachung des Hinweises auf die Patenterteilung: **30.09.2026**
(21) Anmeldenummer: 23218159.4
(22) Anmeldetag: 19.12.2023
(51) Int. Cl.: A61L 27/32, A61L 27/58

(54) **MODIFIZIERTES KNOCHENERSATZMATERIAL**
MODIFIED BONE SUBSTITUTE MATERIAL
MATÉRIAU DE REMPLACEMENT OSSEUX MODIFIÉ

(43) Veröffentlichungstag der Anmeldung: 25.06.2025
(73) Patentinhaber: Haas, Andreas, 69126 Heidelberg (DE)
(72) Erfinder: Haas, Andreas, 69126 Heidelberg (DE); Aydin, Osman, 69126 Heidelberg (DE)
(74) Vertreter: Wunderlich & Heim Patentanwälte PartG mbB

(56) Entgegenhaltungen:
- EP-A1- 3 777 904
- EP-A2- 3 756 624
- JP-A- 2021 029 750
- US-A1- 2020 129 662

## Beschreibung

Die Erfindung betrifft ein Verfahren zum Modifizieren eines Knochenersatzmaterials oder einer Oberflächenbeschichtung eines Implantats zu einem Produkt mit osteotropen Eigenschaften aus einem festen, mikroporösen Ausgangsmaterial, welches im Wesentlichen Hydroxylapatit (Ca₅[OH|(PO₄)₃] aufweist.

Im Rahmen der Erfindung können unter den angeführten Ausgangsmaterialien auch Materialien verstanden werden, welche einen zu den Ausgangsmaterialien analoge Struktur aufweisen, jedoch synthetisch hergestellt sind.

Viele zurzeit verwendete Knochenersatzmaterialien aus natürlichem Knochen, sowohl autolog, allogen als auch xenogen, Korallen, Algen oder auch vollsynthetisch hergestellte Hydroxylapatit-Knochenersatzmaterialien, welche als Knochenimplantat beziehungsweise als Knochenersatzmaterial im Rahmen von Knochenaugmentationen verwendet werden, haben nur osteokonduktive Eigenschaften. Dies bedeutet, dass die bisherigen Calciumphosphat- oder Hydroxylapatit-Knochenersatzmaterialien zwar eine geeignete biokompatible Oberfläche zum direkten Aufwachsen von Knochengewebe auf der Implantatoberfläche bieten, jedoch stimulieren sie nicht direkt die Knochenneubildung in der direkten Knochenumgebung des Implantats.

Hydroxylapatit oder Calciumphosphatmaterialien sind grundsätzlich nur osteokonduktiv. Sie erlauben also das Aufwachsen von Knochen und dienen als Leitstruktur, stimulieren selbst aber nicht die Proliferation oder Ausdifferenzierung von knochenbildenden Zellen, wie Osteoblasten oder deren Vorläufern. Unter direktem Aufwachsen im Sinne der Erfindung kann insbesondere das Aufwachsen ohne "Zwischen-Gewebeschicht" zwischen Implantatoberfläche und Knochengewebe verstanden werden.

Um eine osteoinduktive Wirkung zu entfalten, werden Knochenersatzmaterialien oder auch organischen Substanzen, wie Kollagene, bisher mit weiteren Proteinen, Peptiden oder anderen Molekülen ergänzt. Beispiele hierfür sind Wachstumsfaktoren wie diverse BMPs, IGF1/2, FGF o.ä. oder auch Serumprodukte. Hierbei besteht die Problematik, dass diese Substanzen häufig unklare Zeiträume im Implantat beziehungsweise am Implantatort verweilen, da sie meist schnell aus dem Implantatlager weggespült oder abgebaut werden. Dadurch liegen die osteoinduktiven Eigenschaften zum einen nur kurzzeitig und nicht wirklich dosierbar vor, zum anderen sind auch systemische Wirkungen im gesamten Organismus möglich, die in der Regel nicht erwünscht sind.

Natürlicher Knochen enthält in der der kristallinen Struktur des Calziumphosphats Spurenelemete bestimmter Ionen, die die anorganische Zusammensetzung der Knochenhartsubstanz letztlich ausmachen. Es ist bekannt, dass diese eingebauten Ionen wie Strontium (Sr), Magnesium (Mg), Mangan (Mn) oder Zink (Zn), einzeln oder kombiniert, eine grundlegende Rolle bei der Knochenentwicklung spielen. Strontium ist in natürlichem Knochen in beträchtlichen Mengen und insbesondere in den Regionen mit erhöhtem Stoffwechselumsatz vorhanden. Sein Vorhandensein ist mit der Steigerung der Osteoklasten-Apoptose und der Verstärkung der osteoblastischen Zellproliferation und Kollagensynthese verbunden, die in der Folge die Knochenbildung aufrechterhalten und die Knochenresorption hemmen.

Strontium ist also eine osteotrope und osteoinduktive Eigenschaft nachgewiesen worden. Daher ist die Erhöhung des Anteils von Strontium als natürliches Spurenelement in einem Knochenersatzmaterial oder einer Implantatoberfläche zur Stimulierung von knochenbildenden Zellen von großem Interesse. Eine solche Erhöhung des Anteils von Strontium verleiht einem Knochenersatzmaterial oder einer Implantatoberfläche die gewünschten osteotropen und osteoinduktiven Eigenschaften.

Gleichermaßen trifft dies auch für die Elemente Zink, Magnesium, Mangan, Gallium und ebenso für Fluor zu, deren Knochenwachstum stimulierende Eigenschaften in der Literatur ebenso beschrieben sind. Es existieren zwei Verfahren zur Herstellung solcher mit Strontium angereicherten Knochenersatzmaterialen. Zum einen wird in der EP 3 777 904 A1 die Herstellung eines Knochenersatzmaterials aus kalkinkrustierenden Algen oder Knochen von Säugetieren als Ausgangsmaterial und weiter in der US 10,688,218 B2 die Herstellung eines Knochenersatzmaterials aus Holz als Ausgangsmaterial beschrieben. Beide Verfahren basieren auf einem bekannten, sehr energieaufwändigen hydrothermalen Verfahren, welches erstmals in US 3,929,971 und EP 230 570 B1 beschreiben wurde. Die Weiterentwicklung der Lehren der Verfahren der erstgenannten Druckschriften EP 3 777 904 A1 und US 10,688,218 B2 besteht dabei in dem zusätzlichen Einbau von Strontium-Ionen in das Kristallgitter durch eine Modifikation des ursprünglichen Verfahrens aus den Druckschriften US 3,929,971 und EP 230 570 B1.

Alle oben genannten Verfahren beschreiben ein technisch und energetisch sehr aufwendiges hydrothermales Verfahren. Dabei wird das Ausgangsmaterial nach Beimengung von chemischen Hilfsstoffen und z.T. hochgiftigen Lösungen in einem geschlossenen Behältnis Temperaturen von bis ca. 230 °C und dem dabei entstehendem Druck über längeren Zeitraum ausgesetzt. Dabei kann es sich um mehrere Stunden, und je nach zugeführter Temperatur, bis hin zu Tagen handeln. Unter diesen Bedingungen wird dann der gewünschte Ionenaustausch von Calcium-Ionen durch Strontium-Ionen innerhalb des Kristallgitters des Ausgangsmaterials erreicht. Die Strontium-Ionen werden bei diesem Verfahren eher homogen, jedoch in nur sehr geringer Menge in das Ausgangsmaterial aufgenommen. An der Oberfläche des so behandelten Ausgangsmaterials steht somit nur wenig Strontium zur Stimulierung der Knochenzellen zur Verfügung. Es handelt sich dabei also nur um den vereinzelten Austausch von Calcium-Ionen durch Strontium-Ionen, von der Umwandlung eines Hydroxylapatits in ein Strontiumapatit kann jedoch nicht die Rede sein.

Trotzdem entfaltet ein so hergestelltes Knochenersatzmaterial eine örtliche osteotrope und osteoinduktive Wirkung, welche hauptsächlich und im Wesentlichen nur im Implantatlager stattfindet. Damit hat ein solches Knochenersatzmaterial lokale, nicht aber systemische Wirkung. Ein weiterer Vorteil ist, dass ein solches Knochenersatzmaterial seine osteoinduktiven bzw. osteotropen Wirkungen über die Gesamtzeit des Verbleibs im Implantatlager entfaltet und erst beendet, sobald es von autochtonem Knochengewebe ersetzt wird. So kann bereits durch geringgradige Erhöhung des Anteils von Strontium eine schnellere und nachhaltigere Knochenheilung eines Knochendefektes erreicht werden.

Der Erfindung liegt daher die **Aufgabe** zugrunde, ein Verfahren zum Modifizieren eines Knochenersatzmaterials oder einer Oberflächenbeschichtung eines Implantats zu einem Produkt mit osteotropen Eigenschaften sowie ein osteotropes Knochenersatzmaterial oder eine osteotrope Oberflächenbeschichtung anzugeben, welches langwirksame, örtlich begrenzte osteotrope und osteoinduktive Eigenschaften aufweisen.

Diese Aufgabe wird erfindungsgemäß durch ein Verfahren zum Modifizieren eines Knochenersatzmaterials oder einer Oberflächenbeschichtung eines Implantats mit den Merkmalen des Anspruches 1 sowie ein osteotropes Knochenersatzmaterial oder eine osteotrope Oberflächenbeschichtung mit den Merkmalen des Anspruches 11 gelöst.

Vorteilhafte Ausführungsformen der Erfindung sind in den Unteransprüchen angegeben.

Entsprechend der Erfindung ist vorgesehen ein Knochenersatzmaterial oder eine Oberflächenbeschichtung eines Implantats zu einem Produkt mit osteotropen Eigenschaften zu modifizieren. Hierbei soll das Produkt aus einem festen, mikroporösen Ausgangsmaterial, welches im Wesentlichen Hydroxylapatit (Ca5[OH|(PO4)3] aufweist, sein.

Bei dem Ausgangsmaterial der Erfindung handelt es sich um kleine poröse Festkörper in Form von ca. 0,2 bis 2,0 mm messenden Granulaten oder größeren Formkörpern, die synthetisch hergestellt oder natürlichen Ursprungs sind. Sie liegen bereits in kristalliner Form makroskopisch sichtbar vor und diese makroskopische Form wird durch die Anwendung des Verfahrens nicht verändert.

Ihre Beschaffenheit dient als Volumengeber und eine Art "Ranggitter" oder "Leitstruktur" für Knochenwachstum und sollte daher auch bei klinischer Anwendung ihre Form beibehalten. Nicht zu Verwechseln ist dieses Material mit den in der Literatur oft beschriebenen Knochenzementen. Diese dienen zur Befestigung von z.B. orthopädischen Prothesen, wie Hüftprothesen oder Schenkelhalsprothesen und weiter mittels "Einspritzen" in frakturierte Wirbelköper zu deren Stabilisierung. Bei Knochenzementen werden Pulver feinster Mahlung nach Zugabe von Lösungen unmittelbar vor der Anwendung am Patienten angemischt und dann in niedriger oder hoher Viskosität (flüssig oder breiig) mit der Prothese eingebracht. Unter einer chemischen exothermen Reaktion mit Wärmebildung binden diese ab und härten unter Ausbildung feinster kristalliner Strukturen aus.

Mit derartigen Knochenzementen werden Spalten oder Passungenauigkeiten zwischen Knochen und der Prothese, z.B. einer Hüftprothese aus Titan, ausgeglichen. Dies führt zum stabilen Sitz der Prothese im Knochen durch die hierdurch erreichte exakte Passung. Weiter können durch sie Hohlräume osteoporotisch frakturierter Wirbelköper ausgefüllt und so weitere Frakturen durch Restabilisierung des Wirbelkörpers verhindert werden.

Derartige Stabilisierung kann man mit Knochenersatzmaterial nicht erreichen, da hier erst Knochenbildung um das Material herum entstehen muss. Das Material selbst gibt also keine Stabilität, wie dies ein Knochenzement vermag. Bei Knochenersatzmaterial, wie hier beschrieben und verwendet, handelt es sich also um kleine Festkörper, deren poröse und interkonnektierende Strukturen genutzt werden, um das Aufwachsen auf und Einwachsen zwischen die granulatartigen Festkörper zu ermöglichen. Das Knochenersatzmaterial wird letztlich vollkommen in den neu gebildeten Knochen inkorporiert und ggf. über die Jahre im Rahmen der natürlichen knöchernen Umbauprozesse resorbiert und durch echten Knochen ersetzt. Ihre kristalline Mikrostruktur und makroskopische Form besteht bereits vor der Anwendung und soll auch nach Anwendung möglichst erhalten bleiben. Anders als bei Knochenzementen bilden sich hier keine neuen Kristalle durch eine chemische Reaktion unter Wärmebildung bei Anwendung am Patienten aus.

Zusammenfassend handelt es sich also bei Knochenzementen um Pulver, die zum Zeitpunkt der Anwendung durch Zugabe von Lösungen zu einer flüssigen bis breiigen Masse vermengt werden und unter chemischer Reaktion mit Wärmeentwicklung sowie unter Ausbildung kleinster kristalliner Strukturen abbinden und aushärten. Sie gleichen Ungleichheiten in der Passung zweier Stoffgrenzen aus oder dienen als sofortbelastbare Stütze bei Wirbelkörperfrakturen. Ein Einwachsen von neuem Knochen ist quasi nicht möglich. Im Gegensatz dazu liegen Knochenersatzmaterialien bereits in kristalliner Form als Granulate vor, reagieren bei Anwendung nicht im Sinne einer chemischen Reaktion und haben keine stabilisierende, sondern lediglich eine volumengebende und konduktive Funktion bei Knochenneubildung.

Nach dem erfindungsgemäßen Verfahren ist es vorgesehen eine Modifizierungsmaterials aus einer festen Quelle für Strontium- Fluor- Magnesium-, Mangan-, Zink und/oder Galliumatomen auf einen durchschnittlichen Durchmesser von maximal 1,0 mm oder kleiner zu kleineren und das zerkleinerte Modifizierungsmaterial auf das Ausgangsmaterial zum Herstellen eines Zwischenproduktes aufzubringen. Anschließend wird das Zwischenprodukt bei einer Behandlungstemperatur unterhalb des Schmelzpunktes des Modifizierungsmaterials und des Knochenersatzmaterials oder der Oberflächenbeschichtung des Implantats für eine Behandlungszeit in einer Einrichtung zum thermischen Behandeln in einer offenen Atmosphäre thermisch behandelt, so dass Feuchtigkeit entweichen kann, um das Produkt herzustellen, wobei teilweise Calcium Atome des Hydroxylapatit durch Atome des Modifizierungsmaterials ersetzt werden. Hierbei weist das Modifizierungsmaterial des Zwischenproduktes eine maximale Feuchtigkeit von 10% auf und die Behandlungstemperatur und die Behandlungszeit liegen in einem Bereich, dessen untere Grenze bei mindestens 400 °C Behandlungstemperatur und mindestens 30 min Behandlungszeit liegt und wobei der Bereich weiter durch die Kombinationen mindestens 500°C Behandlungstemperatur und mindestens 10 min Behandlungszeit, mindestens 600°C Behandlungstemperatur und mindestens 7 min Behandlungszeit, mindestens 700°C Behandlungstemperatur und mindestens 5 min Behandlungszeit, mindestens 900°C Behandlungstemperatur und mindestens 3 min Behandlungszeit sowie mindestens 1150°C Behandlungstemperatur und mindestens 2 min Behandlungszeit definiert ist. Abschließend wird das Produkt von nicht eingebauten Modifizierungsmaterial durch mechanische, chemische oder physikalische Reinigungsverfahren gereinigt.

Mit dem erfindungsgemäße Verfahren, ist es erstmal gelungen Strontium-; Fluor-; Magnesium-, Mangan-, Zink- und/oder Gallium-Ionen entsprechend in die dem Knochenwachstum zugewandte Oberfläche des Apatits, insbesondere des Hydroxylapatits, einzubauen und nicht lediglich anzulagern oder im gesamten Festkörper in geringer Konzentration zu verteilen, und somit gerade in der wichtigen Anfangsphase des Knochenwachstums der ersten Tage und Wochen direkt an der Oberfläche des Knochenersatzmaterials in erhöhter Konzentration zur Verfügung zu stehen. Bei einer rein oberflächlichen Anlagerung wie beispielsweise aus JP 2021029750 bekannt, wo eine Kristallzüchtung des Strontiumapatits auf einer Oberfläche beschrieben wird, könnte ein Ablösen der angelagerten Substanzen auftreten. Bei einer gleichmäßigen Verteilung im ganzen Festkörper, wie nach dem bekannten hydrothermalen Verfahren der EP 3 777 904 A1 ist die Verteilung der Ionen homogen und die Konzentration an der Oberfläche nur sehr gering. Durch das erfindungsgemäße Verfahren ist es jedoch möglich, die oben angesprochenen Ionen, die osteotrope, also osteoinduktive, antiresorptive und/oder dem Knochengewebsaufbau förderliche Eigenschaften aufweisen, derart mit der entstehenden Apatitstruktur zu verbinden, dass diese nicht ohne weiteres herausgelöst werden können. Dies ist nach dem beschriebenen Verfahren der Fall, da selektiv die mit den Strontium-; Fluor-; Magnesium-, Mangan-, Zink- und/oder Galliumquellen in Kontakt gekommenen Oberflächen des Apatits, insbesondere des Hydroxylapatits, in ihrer chemischen Zusammensetzung verändert werden

So entstehen beim erfindungsgemäßen Verfahren z.B. bei der Verwendung von Strontiumfluorid als Strontium-Quelle im Kontaktbereich an der Oberfläche eines Hydroxylapatits nach Wärmebehandlung nach beschriebenem Verfahren oberflächliche "Inseln" und "bandartige Areale" aus Strontiumphosphat, welche fest in der Oberfläche des Hydroxylapatits integriert sind. Diese "Inseln" stellen Areale dar, in denen durch IonenAustausch eine Umwandlung von Calziumphosphat in Strontiumphosphat stattgefunden hat. In diesen klar demarkierten Arealen wurde somit durch Energiezufuhr Hydroxylapatit in das bioaktive Strontiumapatit umgewandelt.

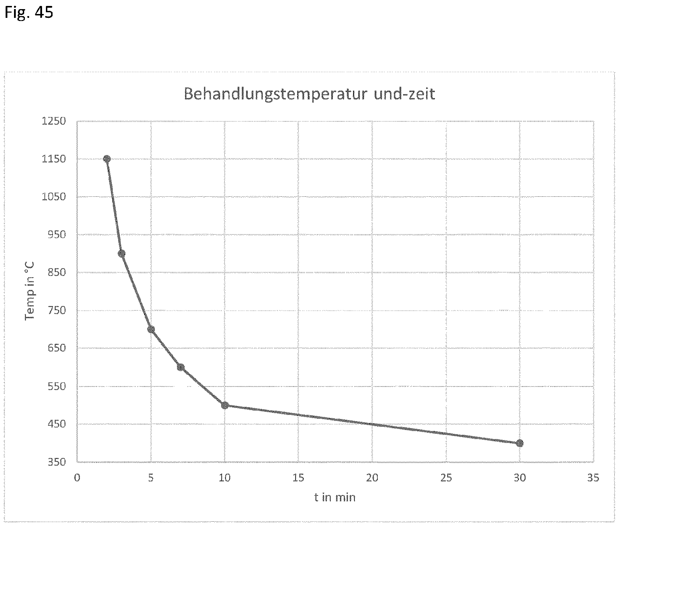

Die Behandlungszeit und die Behandlungstemperatur stehen miteinander in Verbindung. Das bedeutet, dass bei einer langen Behandlungszeit eine geringere Behandlungstemperatur gewählt werden kann als bei einer kurzen Behandlungszeit. Nach der Erfindung wird diese physikalisch-chemische Beziehung zwischen Zeit und Energiezufuhr dadurch verdeutlicht, dass die erfindungsgemäße Behandlungszeit und Behandlungstemperatur mittels eines Bereiches angegeben ist, der durch seine Grenzen definiert ist. Die unterste beschriebene Kombination beträgt hierbei 400°C Behandlungstemperatur und mindestens 30 min Behandlungszeit. Wird die Behandlungstemperatur erhöht, kann die Behandlungszeit verringert werden. Dies bedeutet, dass die Erfindung nicht erfolgreich bei einer Behandlungstemperatur von 400 °C und einer Behandlungszeit von nur 20 min ausgeführt werden kann. Weitere Punkte zum Definieren der möglichen Kombinationen sind mindestens 500°C Behandlungstemperatur und mindestens 10 min Behandlungszeit, mindestens 600°C Behandlungstemperatur und mindestens 7 min Behandlungszeit, mindestens 700°C Behandlungstemperatur und mindestens 5 min Behandlungszeit, mindestens 900°C Behandlungstemperatur und mindestens 3 min Behandlungszeit sowie mindestens 1150°C Behandlungstemperatur und mindestens 2 min Behandlungszeit. Dies ist in der Figurenbeschreibung zu Figur 45 noch weiter erläutert. Unter 400°C findet keine oder so gut wie keine Reaktion statt. Oberhalb von 1150°C schmilzt, je nach gewähltem Modifizierungsmaterial, dieses bereits, bzw kommt es zur Sinterung.

Grundsätzlich kann das Aufbringen des zerkleinerten Modifizierungsmaterials auf das Ausgangsmaterial beliebig erfolgen. Es wurde jedoch erkannt, dass verschiedene Verfahren hierbei besonders gute Ergebnisse liefern. So kann das zerkleinerte Modifizierungsmaterial zumindest teilweise in einer Flüssigkeit, bevorzugt Wasser, gelöst werden und auf das Ausgangsmaterial aufgebracht werden, wobei eine anschließende Trocknung zum Entfernen der Flüssigkeit vorgesehen ist. Die Flüssigkeit dient somit als Trägermedium für des Modifizierungsmaterial. Nach dem Trocknen bleibt das Modifizierungsmaterial auf dem Ausgangsmaterial übrig.

Eine andere Möglichkeit ist, dass das zerkleinerte Modifizierungsmaterial mit geringen Mengen einer Flüssigkeit versetzt wird, um eine schlammartige Konsistenz zu erzeugen und dieser Schlamm auf das Ausgangsmaterial aufgetragen wird. Auch hier wird die Flüssigkeit als Trägermedium verwendete, wobei durch das Herstellen einer schlammartigen Konsistenz, ein ausreichend guter Auftrag auf das Ausgangsmaterial erreicht wird.

Sofern eine Flüssigkeit eingesetzt wird, um das Modifizierungsmaterial auf Ausgangsmaterial aufzutragen, kann es hilfreich sein, dass vor dem thermischen Behandeln des Zwischenproduktes überschüssiges zerkleinertes Modifizierungsmaterial mittels physikalischer Schritte entfernt wird. Es hat sich gezeigt, dass hier durch bessere Ergebnisse im Endprodukt erreicht werden können.

Es ist aber nicht zwingend notwendig eine Flüssigkeit zur Hilfe zu nehmen, um das Modifizierungsmaterial auf das Ausgangsmaterial aufzutragen. Es ist auch möglich, dass zum Aufbringen des zerkleinerten Modifizierungsmaterials auf das Ausgangsmaterial das Ausgangsmaterial im zerkleinerten Modifizierungsmaterial eingebettet wird und derart thermisch behandelt wird. Alternativ kann auch auf das Ausgangsmaterial trockenes zerkleinertes Modifizierungsmaterial aufgetragen werden.

Vorzugsweise werden als Quellen für Strontium, Fluor, Magnesium, Mangan, Zink und/oder Galliumatome die jeweiligen Stoffe selbst oder chemische Verbindungen, vorzugsweise deren Salze, verwendet werden, die diese Atome aufweisen, insbesondere ausgewählt aus: Strontiumfluorid, Magnesiumfluorid, Zinkfluorid, Mangan, Galliumoxid. Hierbei kann ein guter Einbau beziehungsweise Austausch der Atome mit den Atomen des Ausgangsmaterial erfolgen.

Vorteilhaft ist es ferner, wenn als Strontium-; Fluor-; Magnesium-, Mangan-, Zink- und/oder Galliumquelle Verbindungen gewählt werden, die für die Verarbeitung ungiftig vorliegen, deren Löslichkeit in einer ungiftigen Lösung ausreichend ist oder die in feiner Pulverform vorliegen können, deren Schmelzpunkte möglichst niedrig sind und idealerweise unter dem Schmelzpunkt des Ausgangsmaterials liegen, deren Restprodukte und Rückstände nach dem Glühvorgang ebenso ungiftig sind und sich gut mittels mechanischen, physikalischen oder chemischen Reinigungsverfahren entfernen lassen. Effektiv haben sich hierbei im Falle des Strontiums ein Salz des Strontiums, nämlich Strontiumfluorid gezeigt. Strontiumfluorid ließ sich in den Versuchen mäßig gut in destilliertem Wasser lösen. Dies konnte durch Wärmezufuhr bis zur Siedetemperatur des destillierten Wassers verbessert werden.

Grundsätzlich kann das thermische Behandeln des Zwischenproduktes beliebig ausgeführt werden. So können Aufheiz- und/oder Abkühlintervalle vorgesehen sein, um die gewünschte Behandlungstemperatur beziehungsweise Raumtemperatur zu erreichen.

Bevorzugt ist es, wenn die Aufheiz- und/oder Abkühltemperaturänderung maximal 10° pro Minute, bevorzugt weniger als 5° pro Minute, beträgt. Es hat sich gezeigt, dass derartige Temperaturänderungen den geringsten Einfluss auf die Langlebigkeit des Endproduktes aufweisen. Schonend für das Material hat sich auch ein Abkühlintervall über eine Stunde bei Raumtemperatur gezeigt.

Mit diesem schonenden Vorgehen kann eine Versprödung oder der Übergang in eine Glasphase der Materialien vermieden werden. Niedrigere Temperaturen sind dabei mit längeren Glühzeiten und geringerem Effekt der der gewünschten Veränderung an der Oberfläche verbunden, wodurch sich jedoch auch die Zusammensetzung des Neuen Materials gut steuern lässt.

Grundsätzlich wird eher ein längeres Erhitzen des zu modifizierenden Materials bei eher niedrigen Temperaturen bevorzugt, um das Ausgangsmaterial schonend zu modifizieren. Es wurde entsprechend der Erfindung festgestellt, dass ein Erhitzen auf 900°C für 30 min bereits hervorragende Ergebnisse liefert, so dass ein noch längeres Erhitzen oder höhere Temperaturen nicht zwingend zu deutlich besseren Ergebnissen führt.

Im Falle der Verwendung von Hydroxylapatit in Form des Knochenersatzmaterials aus Rinderknochen oder aus Algen als Ausgangsmaterial und Strontiumfluorid als Strontiumquelle haben sich Temperaturen von 400°C bis 1150°C und einer Glühdauer von mindestens 2 min bis max. 300 min erwiesen, um das gewünschte Ergebnis, nämlich eine Umwandlung von Hydroxylapatit (Calziumphosphat) in das gewünschte Strontiumapatit (Strontiumphosphat) im direkten Kontaktbereich mit der Ionen-Quelle Strontiumfluorid zu erreichen, ohne dabei eine Schädigung des Ausgangsmaterials zu verursachen.

Es ist aber auch möglich das Zwischenprodukt direkt in eine Behandlungseinrichtung einzubringen, welche im Wesentlichen die Behandlungstemperatur aufweist. Besonders gute Ergebnisse konnten entsprechend den Untersuchungen, welche der Erfindung zugrunde liegen, erreicht werden, wenn die die Behandlungstemperatur unter dem Schmelzpunkt des Ausgangsmaterials und der Ionen-Quelle lag.

Als Knochenersatzmaterial wird ein Ausgangsmaterial, welches im Wesentlichen Hydroxylapatit (Ca₅[OH(PO₄)₃]) aufweist, verwendet; weitere bekannte Knochenersatzmaterialien sind Calciumphosphat, Tricalciumorthophosphat oder Tricalciumphosphat, Calciumhydroxid, Calciumoxid, Aragonit, Kalzit in Form von gebrannten, ungebrannten und/oder chemisch aufbereiteten biologischen Skeletten oder Wirbeltier- oder Säugetierknochen nach oder vor pyrolytischer oder chemischer Mazeration.

Aragonit oder Kalzit, z.B. von kalkinkrustiernden Algen, können in gebrannter, ungebrannter und/oder chemisch aufbereiteter Form eingesetzt werden. Bei dem Verwenden von Wirbeltier- oder Säugetierknochen werden diese vorteilhafterweise zuvor einer pyrolytischen oder chemischen Mazeration, das heißt, dem Entfernen von immunogenem Material unterzogen.

Zu bevorzugen ist es, wenn das Ausgangsmaterial, im Falle organischen Ursprungs, vor der Behandlung gemäß der Erfindung bereits einer pyrolytischen Behandlung und/oder einer chemischen Reinigung unterzogen und somit von organischen Bestandteilen befreit wurde. Für synthetisch hergestellte Materialen trifft dies nicht zu.

Ferner betrifft die Erfindung ein osteotropes Knochenersatzmaterial oder eine osteotrope Oberflächenbeschichtung eines Implantats, welche nach dem erfindungsgemäßen Verfahren hergestellt wurden. Hierbei sind Calziumatome des Hydroxylapatits durch Strontium-, Fluor-, Magnesium-, Mangan-, Zink- und/oder Galliumatome teilweise ersetzt.

Die osteotrope und osteoinduktive Oberfläche eines solchen Knochenersatzmaterials oder Implantats, welche/s im Wesentlichen Apatit aufweist, zeigt an der dem Knochenwachstum zugewandten Oberfläche inselartige oder bandartige, scharf begrenzte Areale von Kristallumwandlungen des Hydroxylapatits in spezifische Kristalle der entsprechenden Ionen der Strontium-; Fluor-; Magnesium-, Mangan-, Zink- und/oder GalliumQuelle. Diese Areale entfalten osteotrope und osteoinduktive Eigenschaften nach dem Implantieren in einen tierischen oder menschlichen Körper. Neben Apatit, insbesondere Hydroxylapatit, können auch geringe Bestandteile von Calziumphosphat insbesondere Tricalziumphosphat, und Calziumcarbonat, insbesondere Calcit oder Aragonit vorhanden sein.

Das gemäß dem erfindungsgemäßen Verfahren hergestellte osteotrope und osteoinduktive Knochenersatzmaterial kann beispielsweise zum Herstellen von formstabilen Blöcken verwendet werden. Da das entstehende osteotrope Knochenersatzmaterial eine granulatartige bis pulverartige Form aufweist, können die formstabilen Blöcke, welche aus diesem hergestellt werden, nach Maß angefertigt und somit auf Knochendefekte angepasst werden. Auch können Oberflächen von Implantaten, ob metallischer oder keramischer Natur, soweit sie Anteile an Apatit, vorzugsweise Hydroxylapatit bzw. Calciumphosphat enthalten, in dieser Weise einer Oberflächenveredelung unterzogen werden, sodass durch die Entstehung einer bioaktiven, osteotropen und osteoinduktiven Oberfläche eine knöcherne Einheilung, also eine sogenannte Osseointegration, des jeweiligen Implantates im Knochen schneller und wahrscheinlicher abläuft. Beispielsweise könnten hydroxylapatitbeschichtete Zahnimplantate aus Titan oder auch Zahnimplantate aus Vollkeramik für eine bessere Einheilung im Kieferknochen einer solchen Wärmebehandlung zum Einbau von knochenzellenstimulierenden Strontium-; Fluor-; Magnesium-, Mangan-, Zink- und/oder Galliumionen unterzogen werden.

Durch die erfindungsgemäße Oberflächenveränderung eines Ausgangsmaterials entfaltet dieses, ob in Form von Knochenersatzmaterial oder in Form von Oberflächen auf Implantaten, eine örtliche osteotrope und osteoinduktive Wirkung, welche hauptsächlich und im Wesentlichen nur im oder am Implantatlager stattfindet. Damit hat das erfindungsgemäße Knochenersatzmaterial keine systemische Wirkung. Ein weiterer Vorteil ist, dass die erfindungsgemäße Oberflächenveränderung eines Ausgangsmaterials, ob in Form von Knochenersatzmaterial oder in Form von Oberflächen auf Implantaten, seine osteotropen und osteoinduktiven Wirkungen über die Gesamtzeit des Verbleibs im Implantatlager entfaltet und erst beendet, sobald es von autochtonem Knochengewebe ersetzt wird. So wird eine schnellere und nachhaltigere Knochenheilung eines Knochendefektes oder das Einheilen eines Implantates im Knochen erreicht.

Ein anderes Anwendungsbeispiel für das erfindungsgemäße osteotrope Knochenersatzmaterial kann die Beschleunigung der Heilung von osteoporotischen, traumatischen und/oder malignombedingten Wirbelbrüchen oder Sinterungsfrakturen nach deren Stabilisierung durch die Anregung der Knochenregeneration sein. Eine weitere Möglichkeit wäre, das erfindungsgemäß hergestellte Material als Ausgangsmaterial für 3-D-Druckverfahren einzusetzen.

Im Folgenden wir exemplarisch das Durchführen des erfindungsgemäßen Verfahrens beschrieben und schließend entsprechende Analyseergebnisse erläutert.

Hierbei wird auch auf die Figuren Bezug genommen. Hierbei zeigt:
- Fig. 1: ein Sekundärelektronenbild einer ersten Ausführung des erfindungsgemäßen Verfahrens;
- Fig. 2: Materialkontraste der ersten Ausführung des erfindungsgemäßen Verfahrens;
- Figuren 3 bis 8: Elementverteilungskarten der ersten Ausführung des erfindungsgemäßen Verfahrens;
- Figuren 9 und 10: Einzelpunkt- und Kleinflächenanalysen der ersten Ausführung des erfindungsgemäßen Verfahrens;
- Fig. 11: ein Sekundärelektronenbild einer zweiten Ausführung des erfindungsgemäßen Verfahrens;
- Fig. 12: Materialkontraste der zweiten Ausführung des erfindungsgemäßen Verfahrens;
- Figuren 13 bis 18: Elementverteilungskarten der zweiten Ausführung des erfindungsgemäßen Verfahrens;
- Figuren 19 und 20: Einzelpunkt- und Kleinflächenanalysen der zweiten Ausführung des erfindungsgemäßen Verfahrens;
- Fig. 21: ein Sekundärelektronenbild einer dritten Ausführung des erfindungsgemäßen Verfahrens;
- Fig. 22: Materialkontraste der dritten Ausführung des erfindungsgemäßen Verfahrens;
- Figuren 23 bis 30: Elementverteilungskarten der dritten Ausführung des erfindungsgemäßen Verfahrens;
- Figuren 31 und 32: Einzelpunkt- und Kleinflächenanalysen der dritten Ausführung des erfindungsgemäßen Verfahrens;
- Fig. 33: ein Sekundärelektronenbild einer vierten Ausführung des erfindungsgemäßen Verfahrens;
- Fig. 34: Materialkontraste der vierten Ausführung des erfindungsgemäßen Verfahrens;
- Figuren 35 bis 42: Elementverteilungskarten der vierten Ausführung des erfindungsgemäßen Verfahrens;
- Figuren 43 und 44: Einzelpunkt- und Kleinflächenanalysen der vierten Ausführung des erfindungsgemäßen Verfahrens; und
- Fig. 45: ein Diagramm zur Verdeutlichung des Berichts der Behandlungstemperatur und -Zeit.

Hierzu wurde Strontiumfluorid in destilliertem Wasser gelöst, wobei das Auslöseverhalten durch Wärmezufuhr bis zur Siedetemperatur des destillierten Wassers verbessert wurde. Anschließend wurde ein Ausgangsmaterials aus Hydroxylapatit in Form der Granulate des Knochenersatzmaterials BioOss der Firma Geistlich sowie Algipore der Firma Dentsply Sirona in die Lösung des Strontiumfluorids eingebracht.

Eine gute Benetzung mit der Strontiumquelle wurde durch Rühren und Erwärmen erreicht. Die Entfernung des Wasserüberschusses erfolgte durch Abschütten des Überschusses über einem Sieb. Weiter erfolgte das Trocknen des so behandelten Ausgangsmaterials durch Verdunstung mittels Einstellens in einen Wärmeofen bei 180°C bis zur ausreichenden Trocknung.

Eine Entfernung der gröbsten Überschüsse der Strontiumquelle wurde durch ein einfaches Sieben erreicht. Das so getrocknete und von groben Überschüssen der Strontiumquelle befreite Material wurde dann einer thermischen Behandlung, die auch als Glühvorgang bezeichent werden kann, ausgesetzt und nach Abkühlen mittels Waschung und Siebung in destilliertem Wasser wiederum von groben und losen Überschüssen und Resten der Strontiumquelle befreit.

Beim Glühvorgang wurde ein Hochtemperaturofen für den Dentalbereich mit einer Brennkammer von ca. 15 x 10 x 17 cm und einem Heizmodul zur Programmierung von Aufheizintervallen und Haltestufen sowie Temperaturregelung verwendet.

Das so vorbehandelte und getrocknete Material wurde in der Brennkammer in einer feuerfesten Keramikschale platziert, die luftdurchlässige Brennkammer geschlossen und das Programm zum Glühvorgang gestartet. Unten analysierte Endprodukte wurden dazu bei Haltetemperaturen von 900°C für 30 min oder 60 min ohne ein Aufheizintervall und ohne definiertes Abkühlintervall mit einer einfachen Abkühlung bei Zimmertemperatur hergestellt. Bei diesen Temperaturen und Glühzeiten konnte eine ausreichend gute Umwandlung des Ausgangsmaterials erreicht werden, wobei zusätzlich, wie bereits beschrieben, die genannten Ionen eingelagert und mit dem Kristallgitter des Apatits verbunden wurden. Materialanalysen zeigten hierbei, dass das Hydroxylapatit (Calziumphosphat) des Ausgangsmaterials an der Oberfläche an genau den Stellen in Strontiumapatit (Strontiumphosphat) umgewandelt wurde, an denen Kontaktbereiche mit dem Strontiumfluorid bestanden hatten. Je geringer die Temperatur des Ofens gewählt wurde, um so langsamer und weniger eindrücklich zeigte sich dieses Phänomen. Diese Tatsache könnte für eine Steuerung der Zusammensetzung des gewünschten Endproduktes genutzt werden.

Zur Analyse wurden die wie beschrieben erstellten Proben in Epoxidharz eingebettet. Da die Proben zur Analyse im Rasterelektronenmikroskop vorgesehen waren, wurden diese nicht leitenden Präparate zunächst mit einer dünnen Kohlenstoffschicht leitend gemacht.

Die Proben wurden in eine Rasterelektronenmikroskop (LEO 440) mit Wolframkathode eingebracht. Bei einer Anregungsspannung von 20 KV wurde bei verschiedenen Strahlströmen Bilder aufgenommen. Die gezeigten SE-Bilder (Sekundärelektronenbilder) stellen Topographiekontraste dar.

Die in den Figuren gezeigten BSE-Bilder (back-scattered-electrons bzw. rückgestreute Elektronen) zeigen Materialkontraste. Je heller ein Bildpunkt im BSE-Bild ist, desto höher ist die mittlere Ordnungszahl des an dieser Stelle zu findenden Materials.

So ist z.B. bei den hier vorliegenden Proben eine Stelle deutlich heller, an der das im Apatit vorhandene Ca in deutlichen Mengen durch Sr substituiert wurde.

Die chemische Analyse im REM erfolgte mit Hilfe eines X-Max 150-Detektors. Es handelt sich um einen SDD-Dekektor, der die analysierbaren Elemente energiedispersiv simultan erfassen kann.

Für die Charakterisierung der Proben wurden zunächst für jede Stelle jeweils Elementverteilungskarten erstellt. Hier wurde mit einer Auflösung von 1024 * 768 Pixeln jeweils eine interessant erscheinende Fläche der Probe "kartiert.

Aufbauend auf diese Elementverteilungskarten wurden dann gezielt an einigen Punkten Einzelanalysen durchgeführt. Diese führt zu Spektren mit sehr guter Statistik, so dass eine chemische Analytik mit aussagekräftigen Daten erzeugt werden konnte. Die in den Figuren gezeigten Elementverteilungskarten wurden selektiv ausgewählt. Zwar wurden auch andere Elemente erfasst, diese stellten sich aber als irrelevant für die hier zu betrachtende Fragestellung heraus.

### Probe Knochenmaterial 0,5h / 900°C

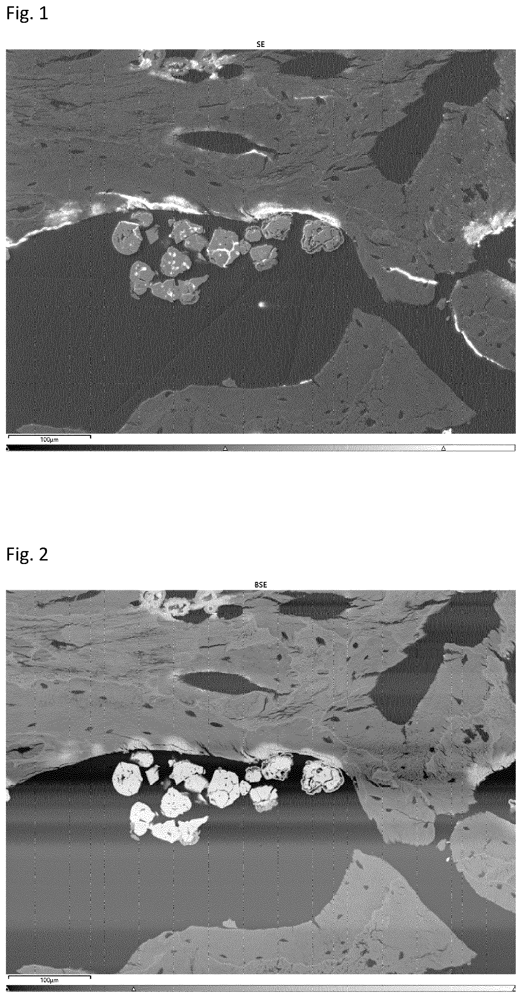

Die in den Figuren 1-10 gezeigten Analysen beziehen sich auf Rinderknochen welches nach dem erfindungsgemäßen Verfahren für 0,5h / 900°C behandelt wurde. Hierbei zeigt Fig.1 ein Sekundärelektronenbild. Man erkennt, dass die polierte Probe fast kein Relief hat. Den Großteil der Probe bildet das hier eingesetzte Knochenmaterial, dass sich in mittleren Grautönen zeigt. Die dunklen Bereiche stammen von den Stellen, an denen sich das Epoxidharz befindet. Die hellgrauen Körner sind in diesem Epoxidharz eingeschlossen. Sie befinden sich in der Nähe zum Knochenmaterial.

Fig. 2 zeigt Materialkontraste. Innerhalb des Knochenmaterials sieht man in den Graustufen klar voneinander abgrenzbare Bereiche, die leichte Unterschiede zeigen. Dieses dürfte daher kommen, dass der Apatit an diesen Stellen leichte Unterschiede in seiner chemischen Zusammensetzung oder in seiner Dichte hat.

Die Körner sind im BSE-Bild deshalb so hell, da sie große Mengen an dem schweren Element Sr enthalten. Auch im oberen Bereich des Bildes sind einige helle Stellen zu sehen, die von Körner kommen, die Sr enthalten.

Bei genauer Betrachtung sieht man aber auch innerhalb des Knochenmaterials diverse Stellen, die hell sind. Dies tritt vor allem dort auf, wo sich die SrF₂-Körner in der Nähe zum Knochenmaterial befinden. Es gibt aber auch helle Stellen, an denen man kein SrF₂ sieht. Hier könnte man vermuten, dass auch hier einmal (vor der Präparation) SrF₂ gewesen ist.

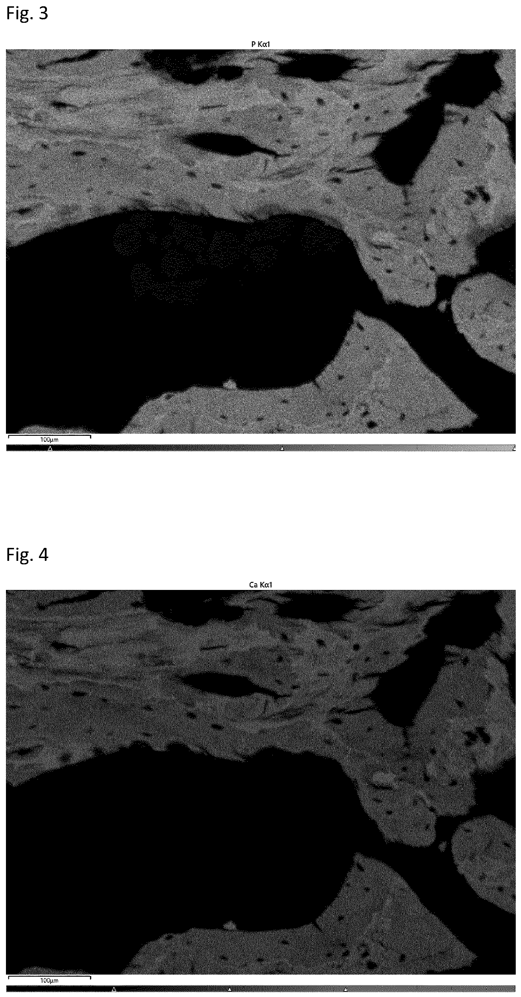

Im Folgenden werden die Elementverteilungskarten erläutert, die in den Figuren 3 bis 8 gezeigt sind. In den Bildern für die Elemente P (Fig 3) und Ca (Fig 4) erkennt man zunächst das Knochenmaterial. Die Konzentrationen der Elemente scheinen hier lokal - wie bei der Diskussion zum BSE-Bild bereits erläutert - zu variieren. Dabei scheint es so zu sein, dass beide Elemente an den jeweils gleichen Stellen ihre höheren und niedrigeren Konzentrationen aufweisen. Es wird davon ausgegangen, dass das Knochenmaterial an diesen Stellen jeweils Variationen in seiner Dichte aufweisen könnte.

Auffällig ist jedoch anderer Sachverhalt. Betrachtet man die in der Elementverteilungskarte die Stellen, an denen im Knochenmaterial das BSE-Bild aufgehellt war, so suggeriert das P-Bild, dass hier weniger Phosphor ist. Im weiteren Verlauf wird man sehen, dass dies nur bedingt richtig ist. Gleichzeitig sieht man im Ca-Bild, dass das Ca an diesen Stellen praktisch nicht mehr sichtbar ist. Hier ist also etwas geschehen.

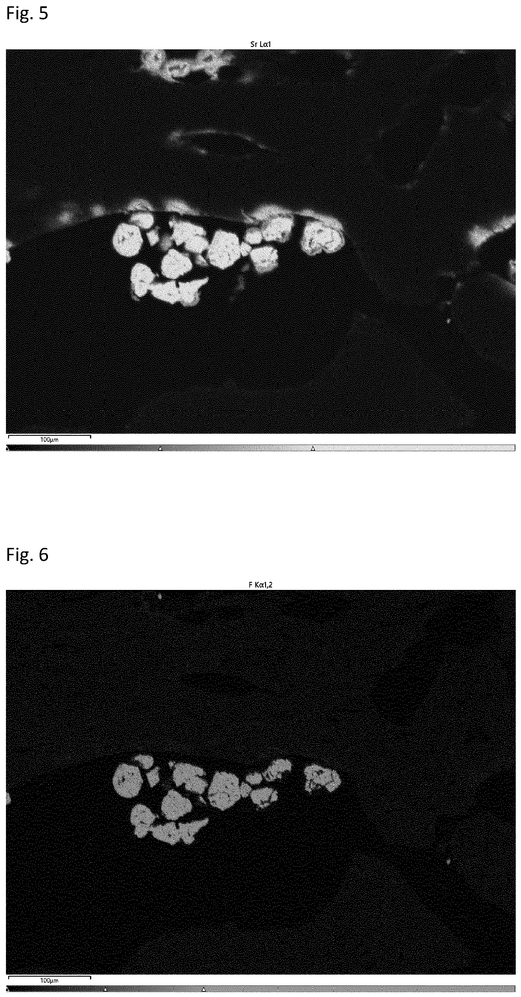

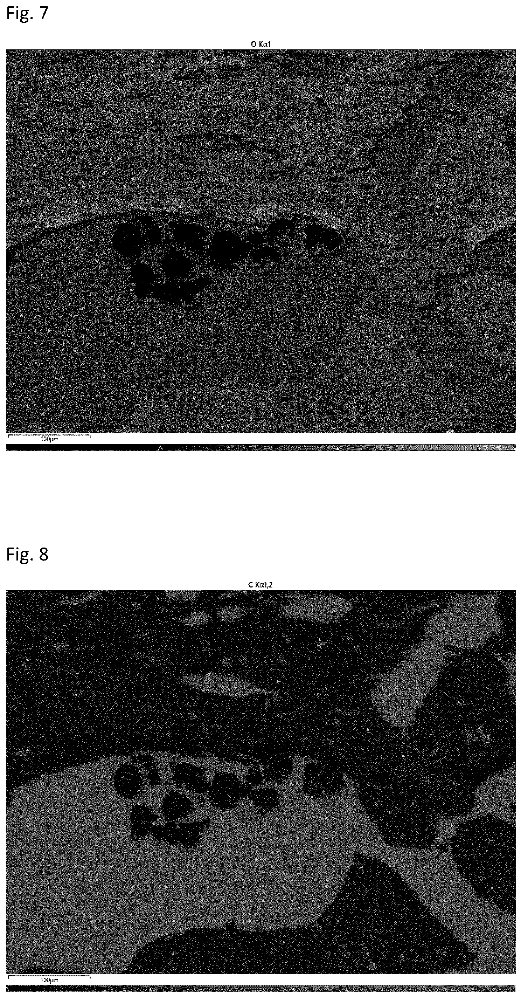

In den Bildern für das Sr (Fig 5) und das F (Fig 6) erkennt man die Position der SrF₂-Körner. Betrachtet man gleichzeitig die Elementverteilung für die Sauerstoff (O) (Fig 7) an diesen Stellen, so sieht man, dass in der Bildmitte einige der Körner einen leichten Saum mit erhöhten Konzentrationen an Sauerstoff aufweisen. Im oberen mittleren Bereich des Bildes ist dies deutlicher ausgeprägt. Dort sieht man kaum noch F, dafür umso mehr O. Da zu Beginn des Experiments das Sr in Form von SrF₂ eingebracht wurde, ist davon auszugehen, dass hier eine Oxidation des Materials während des Versuchs stattgefunden hat. Fig. 8 zeigt eine Elementverteilungskarten für Kohlenstoff (C).

Weiterhin kann man beobachten, dass an Stellen, an denen im Knochenmaterial die Werte für Ca und P niedriger sind, deutliche Konzentrationen an Sr vorhanden sind. Dies sind vor allem die Stellen, die - wie schon erwähnt - räumlich dicht bei den SrF2-Körnern bzw. deren sauerstoffreichen Umwandlungsprodukten zu finden sind. Vor allem diese Stellen gilt es, im Nachgang mit Einzelpunktanalysen zu dokumentieren und näher zu charakterisieren.

### Einzelpunktanalysen

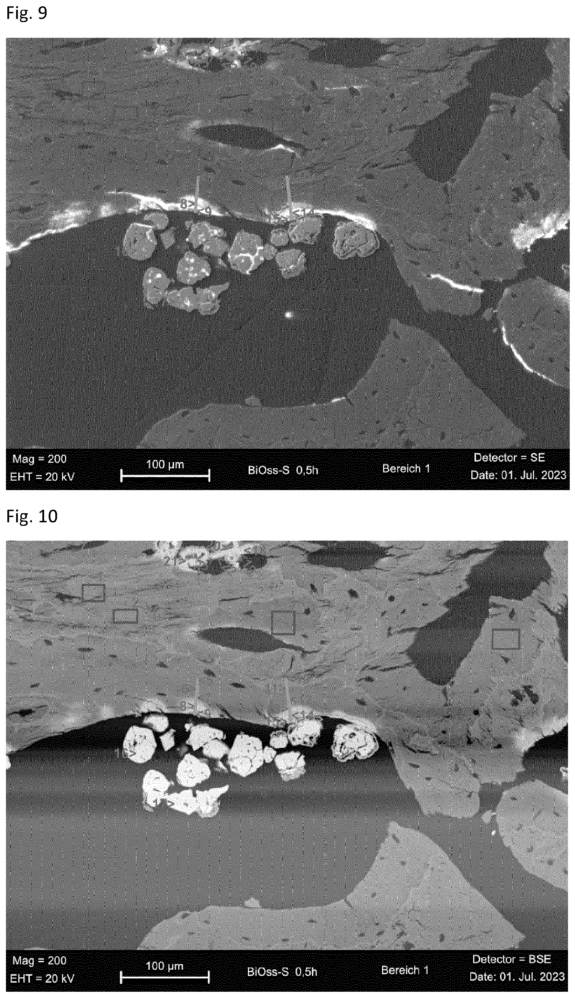

Mit Hilfe von Einzelpunkt- und Kleinflächenanalysen, wie sie in den Figuren 9 und 10 gezeigt sind, wurden nun die sich aus den Elementverteilungskarten ergebenden Befunde detaillierter untermauert.

Für diese Probe zeigen die Punkte 1 bis 4 (kleine Flächenanalysen), dass es sich um "normale" Apatite mit Ca und P handelt. Dazu kommen deutliche Mengen an F und etwas Cl. Das Sr ist im Bereich der Nachweisgrenze.

Das gilt auch für Analysenpunkt 5, der den Anfang eines kleinen Profils bis zu Punkt 9 darstellt. Dabei nimmt der Gehalt an Sr deutlich zu, während der Wert für Ca ebenso sinkt. Am letzten Punkt des Profils (Punkt 9) findet man 71 Gew.-% SrO. Betrachtet man die atomaren Verhältnisse, so scheint es so zu sein, dass der Gehalt von P in etwa konstant bleibt. Ebenso scheint es so zu sein, dass die Summe von Ca und Sr in etwa gleich bleibt. Diese könnte bedeuten, dass man auch bei den Sr-reichen Bereichen eine Phase hat, die stöchiometrisch dem Apatit entspricht, bei dem aber sukzessive das Ca durch Sr ersetzt wird.

Ein weiteres Profil (Punkte 10 bis 15) zeigt ein deckungsgleiches Verhalten.

Analyse 17 zeigt ein nahezu ideales Körnchen von SrF₂, während die Analyse 16 bereits eine gewisse Oxidation von SrF₂ nahelegt.

Die Analysenpunkte 18 bis 23 zeigen SrF₂-Körner, die unterschiedlich stark umgewandelt sind. Dabei kann nicht gesagt werden, um was es sich hier handelt. Die Analysenpunkte 20 und 21 zeigen z.B. deutlich mehr Sauerstoff, als es die bekannten zweiwertigen Kationen Ca und Sr normalerweise aufweisen sollten.

### Probe Knochenmaterial 1,0h / 900°C

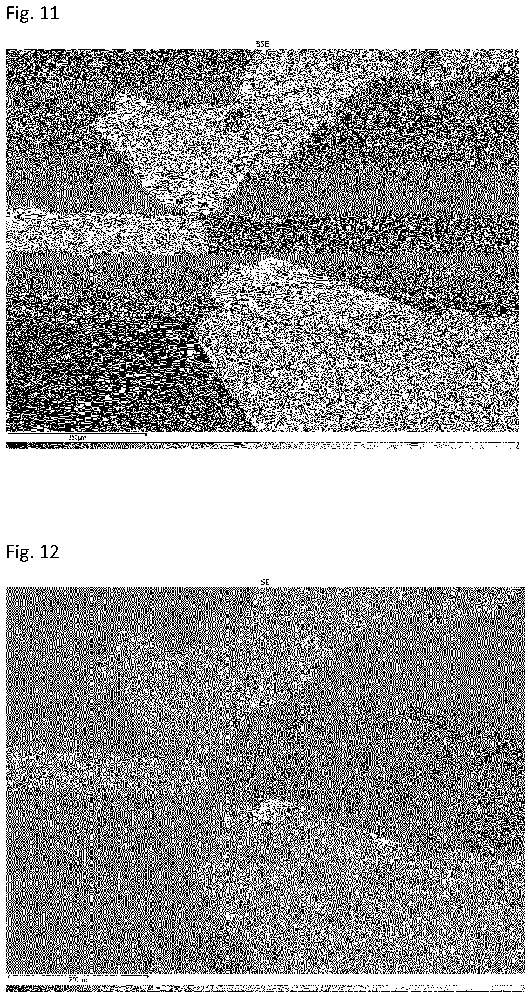

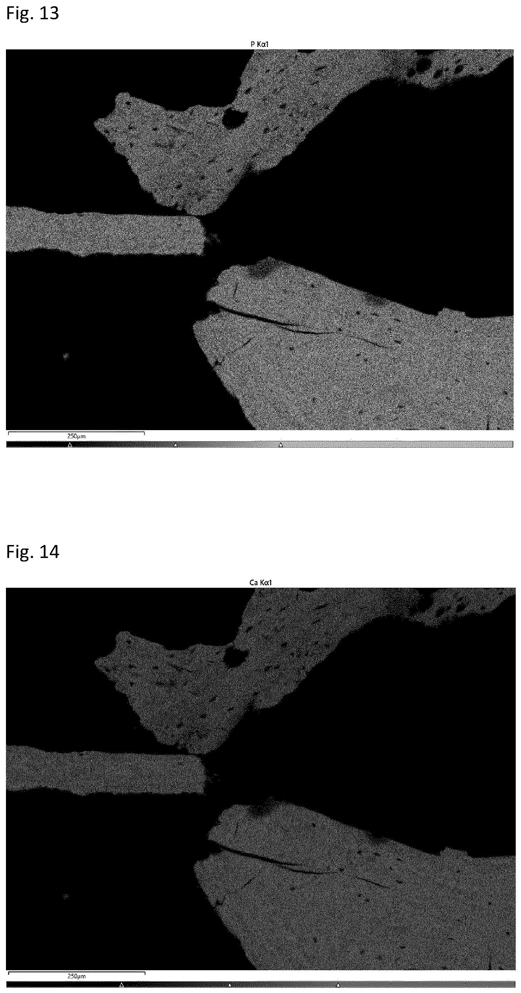

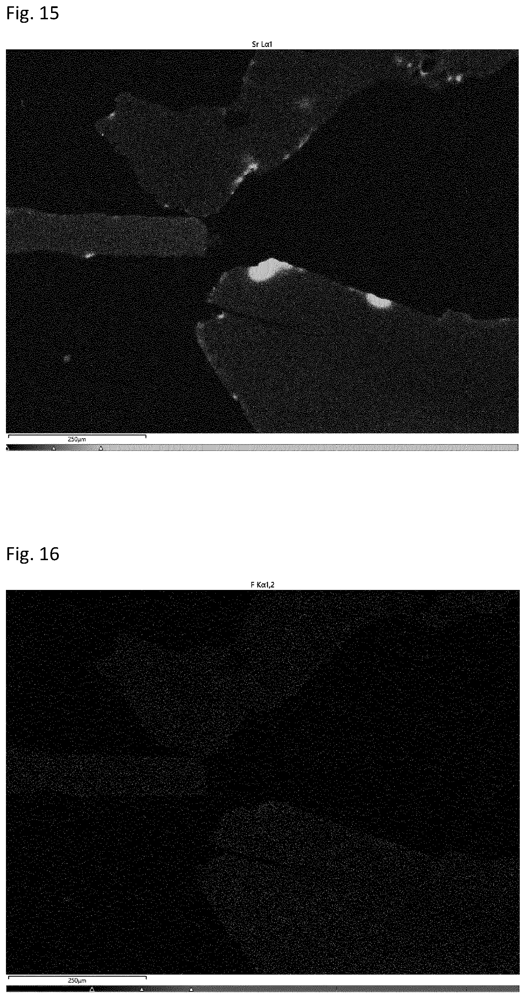

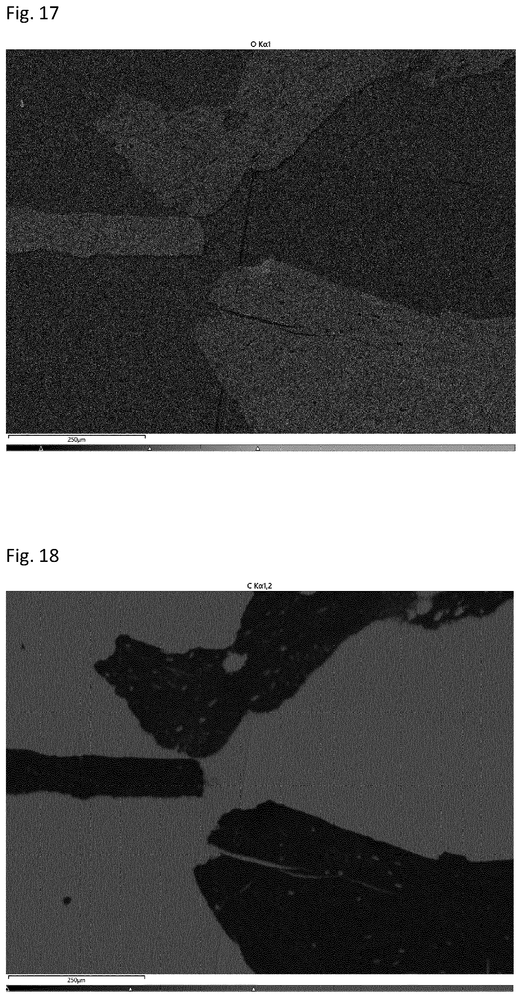

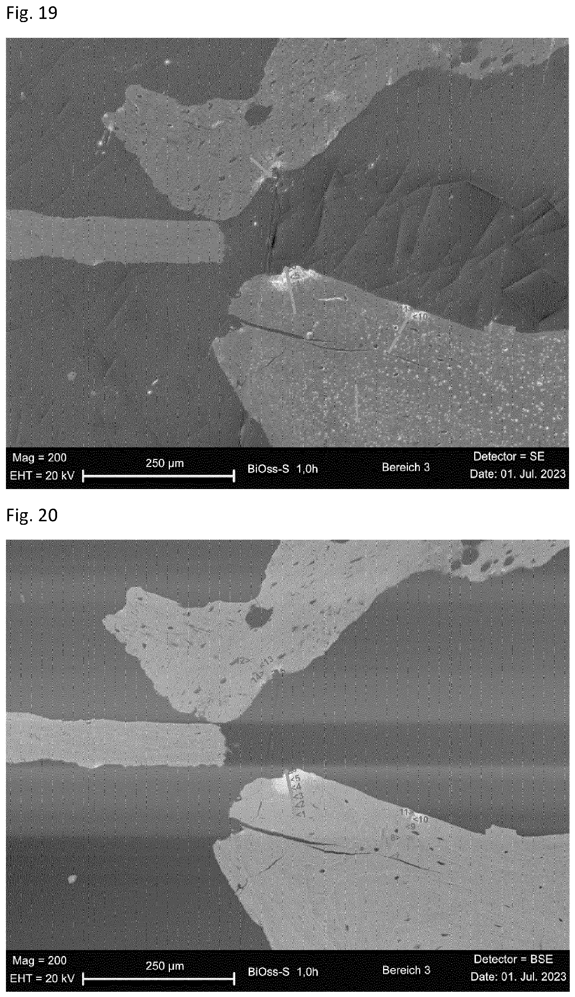

Die in den Figuren 11-20 gezeigten Analysen beziehen sich auf Rinderkonochen, welches nach dem erfindungsgemäßen Verfahren für 1h / 900°C behandelt wurde. Hierbei zeigen die Figuren jeweils die analogen Analysen wie die zuvor beschriebenen Figuren.

Bei der Probe, die 1 Stunde prozessiert wurde, sind die Verhältnisse nicht viel anders als bei der Probe, die nur eine halbe Stunde behandelt wurde. Es wurden jedoch weniger verbliebene Körner von SrF₂ gefunden. Dort, wo es zu Reaktionen mit Einbau von Sr gekommen ist, scheinen diese etwas weiter einzudringen.

Der in den Figuren abgebildete Bereich der Probe enthält kein reliktisches SrF₂.

Sehr eindrucksvoll kann man aber in der Elementverteilungskarte von Sr sehen, dass deutliche Mengen an SrF₂ vorhanden gewesen sein müssen. Sie scheinen aber den Versuch nicht überstanden zu haben und nur massive Konzentrationen von Sr im Apatit hinterlassen zu haben.

Die drei angefertigten Einzelpunktprofile (von 1 bis 6, von 7 bis 11 und von 12 bis 16) zeigen jeweils sehr deutlich den Anstieg der Konzentration von Sr zum Rand hin.

Der Gehalt an C scheint in diesen Profilen ebenfalls zum Rand hin leicht zuzunehmen. Dieses Verhalten war auch bei den vorherigen Stellen schon angedeutet worden, ist aber an dieser Stelle am deutlichsten zu bemerken.

### Probe Algenmaterial 0,5h / 900°C

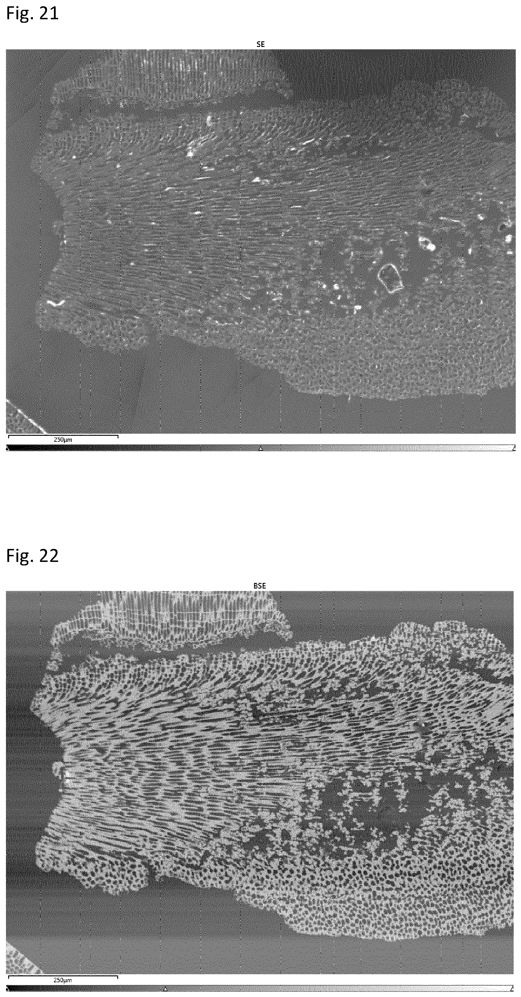

Die in den Figuren 21-32gezeigten Analysen beziehen sich auf Algenmaterial, welches nach dem erfindungsgemäßen Verfahren für 0,5h / 900°C behandelt wurde.

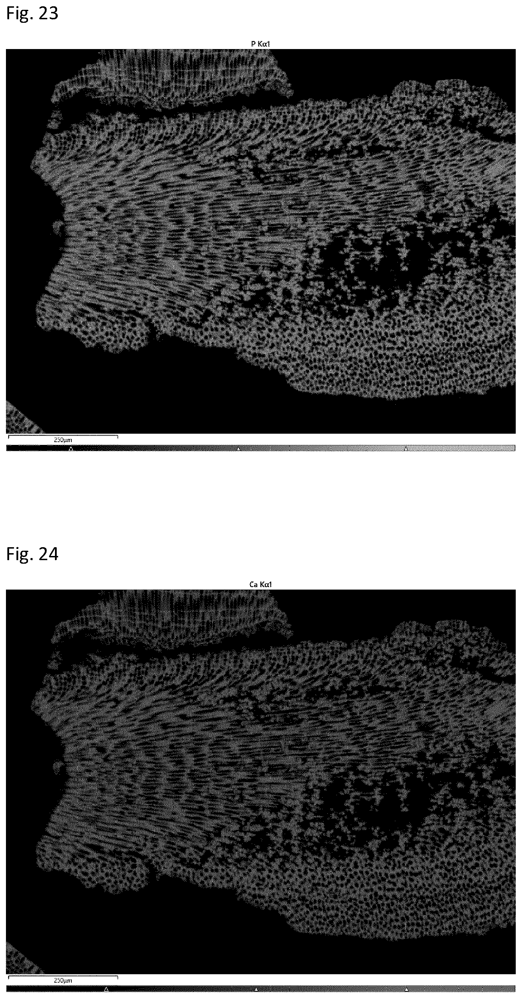

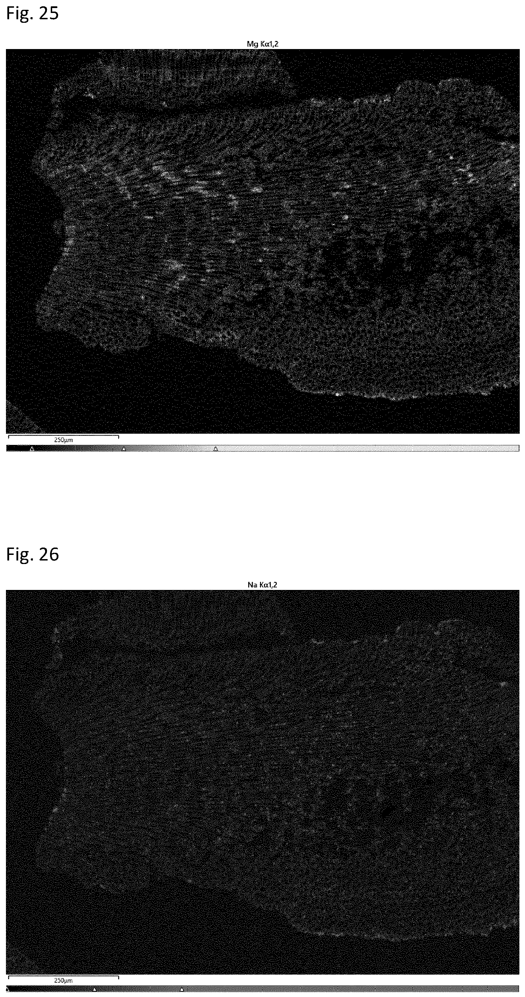

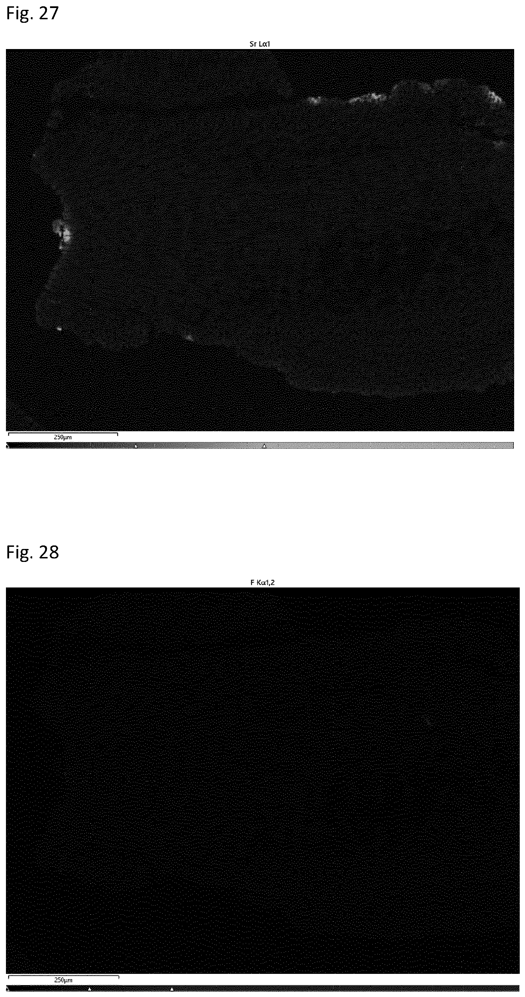

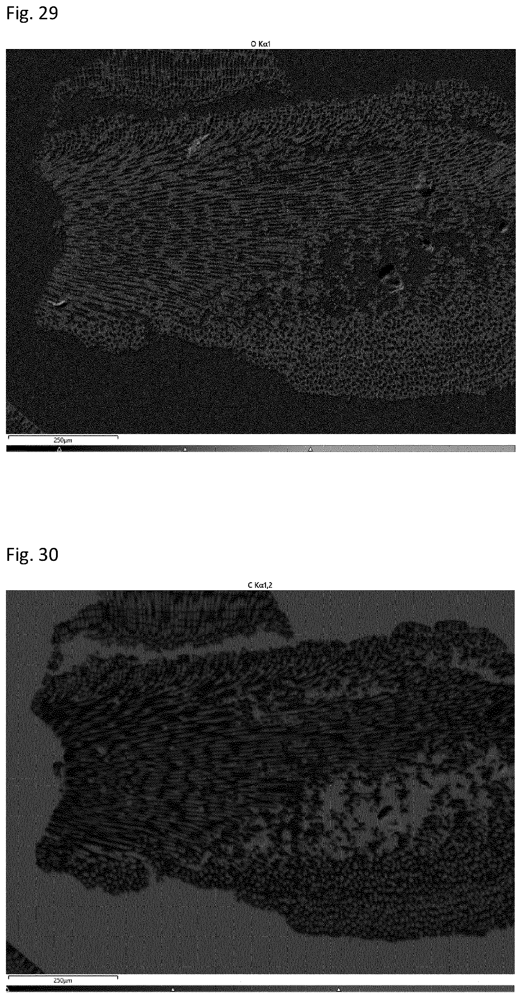

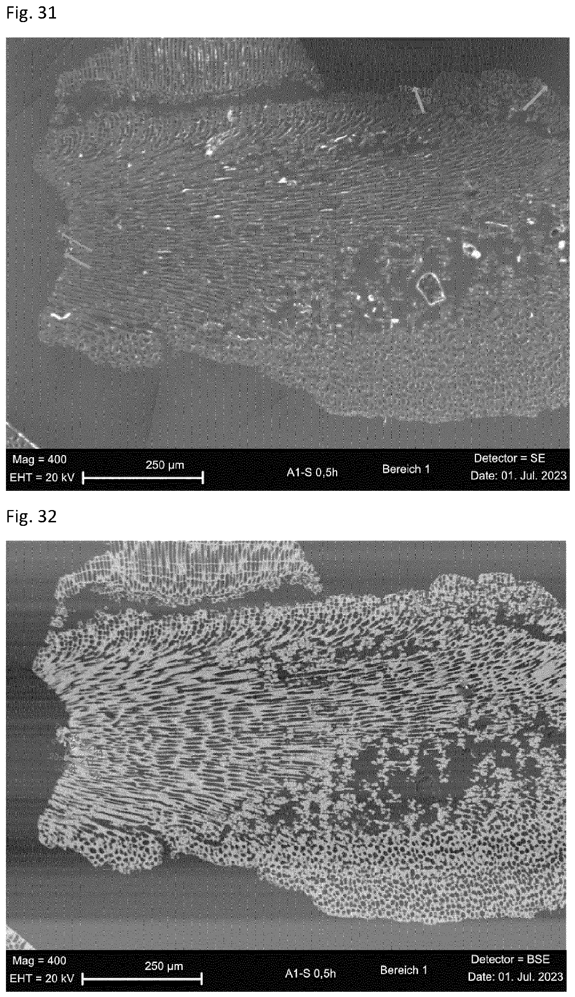

Fig.21 zeigt ein Sekundärelektronenbild und Fig. 22 wiederum Materialkontraste. In Fig. 23 ist eine Elementverteilungskarten für P, in Fig. 24 eine Elementverteilungskarten für Ca, in Fig. 25 eine Elementverteilungskarten für Mg, in Fig. 26 eine Elementverteilungskarten für Na, in Fig. 27 eine Elementverteilungskarten für Sr, in Fig. 28 eine Elementverteilungskarten für F, in Fig. 29 eine Elementverteilungskarten für O, in Fig. 30 eine Elementverteilungskarten für C dargestellt. Figuren 31 und 32 zeigen wiederum Einzelpunkt- und Kleinflächenanalysen.

Das Algenmaterial dieser Probe besteht aus einem Ca-Phosphat.

Während man im BSE-Bild nur angedeutet chemische Inhomogenitäten erkennen kann, sind die Elementverteilungskarten deutlich aussagekräftiger.

Man erkennt zum einen, dass es im Ca-Phosphat lokale Anreicherungen von Mg gibt. Hierbei dürfte das Mg als Ersatz für das Ca eingebaut worden sein. Auffällig ist auch, dass das Mg teilweise auch Anreicherungen am Rand der Körner aufweist. Dies weist auf eine deutliche Mobilisierung des Mg während des Heizprozesses hin. Es könnte durchaus sein, dass man diese Mobilisierung erst erreicht, wenn man sich dem Schmelzpunkt des Materials nähert. Aber hier kann keine sichere Aussage getroffen werden.

Ähnliche Aussagen kann man für das Na machen, das ebenfalls am Rand des Algenmaterials seine höchsten Konzentrationen aufweist.

Deutlich ist in der Elementverteilungskarte auch die Anreicherung von Sr am Rand des Algenmaterials zu erkennen. Es wurden 4 Profile (1 bis 5, 8 bis 11, 12 bis 15 und 16 bis 20) analysiert. Alle diese Profile zeigen einen deutlichen Anstieg des Sr zu Lasten des Ca.

Das Element Chlor spielt in diesem Algenmaterial keine Rolle. Hingegen ist das Fluor prominent vertreten.

### Probe: Algenmaterial 1,0h / 900°C

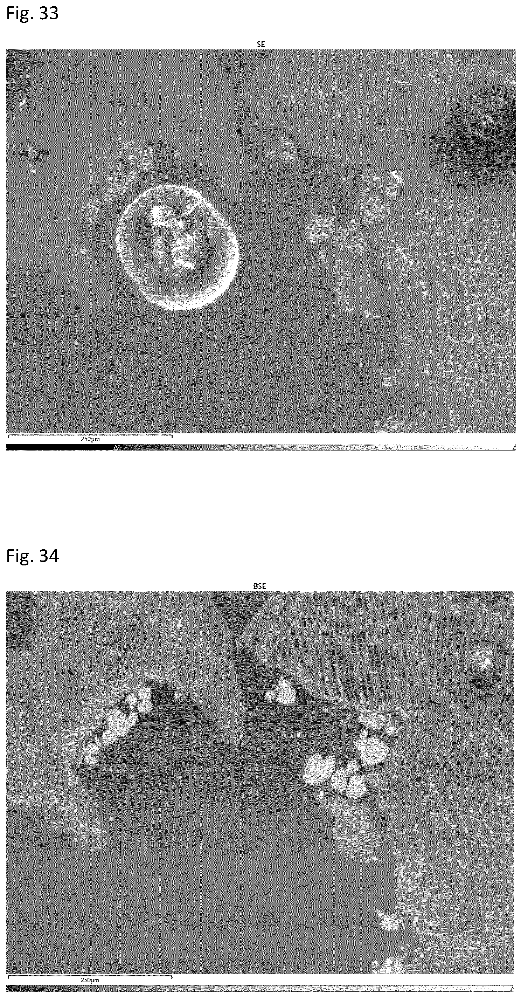

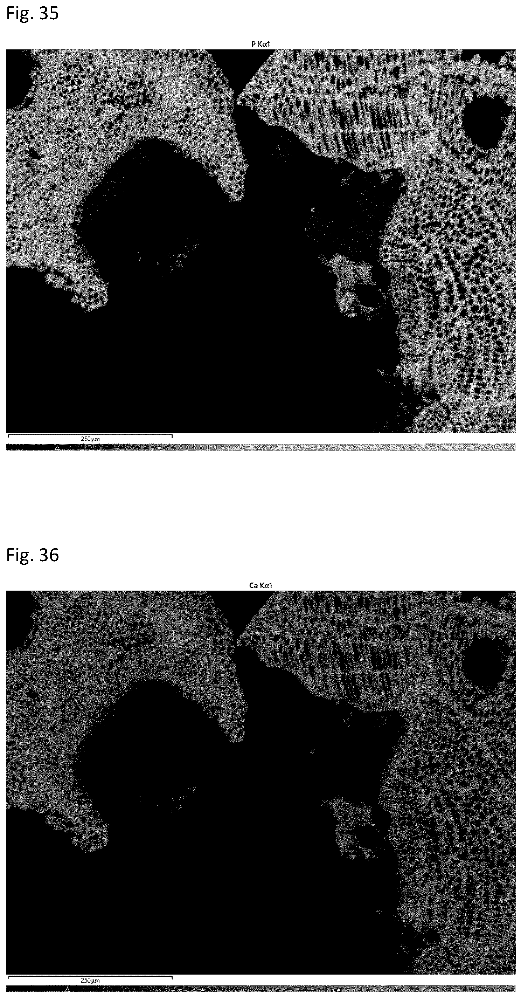

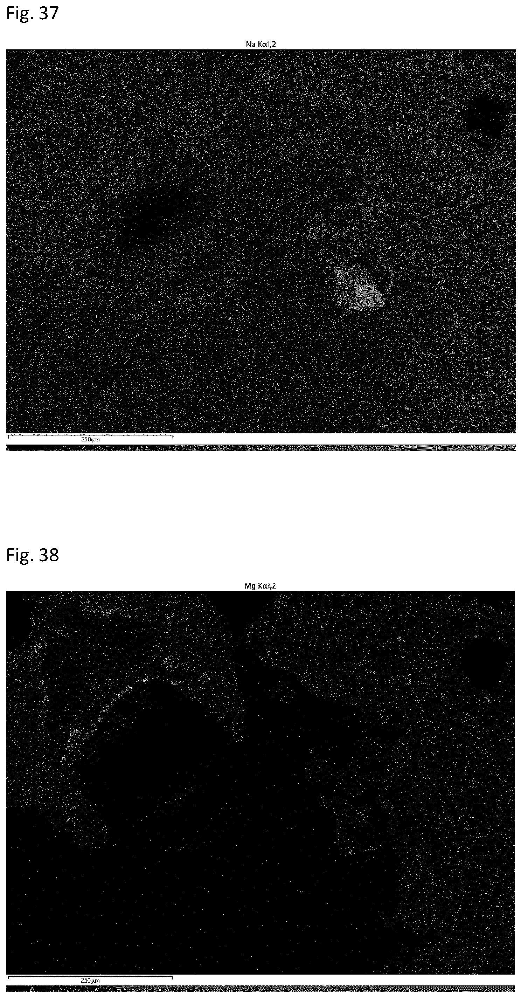

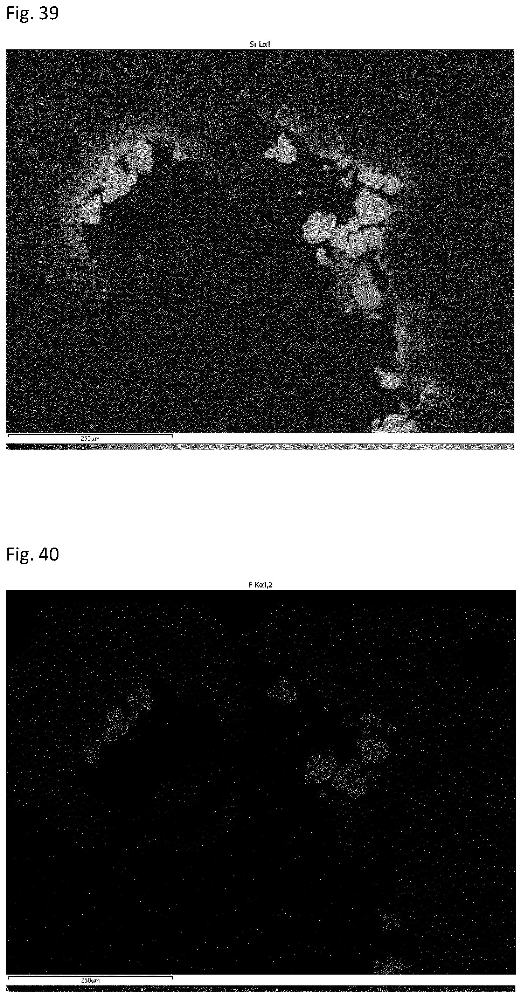

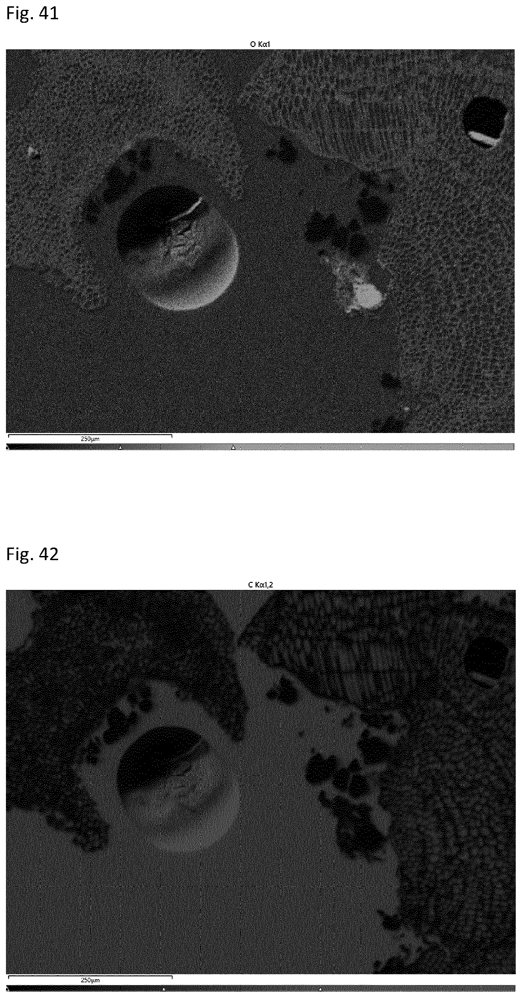

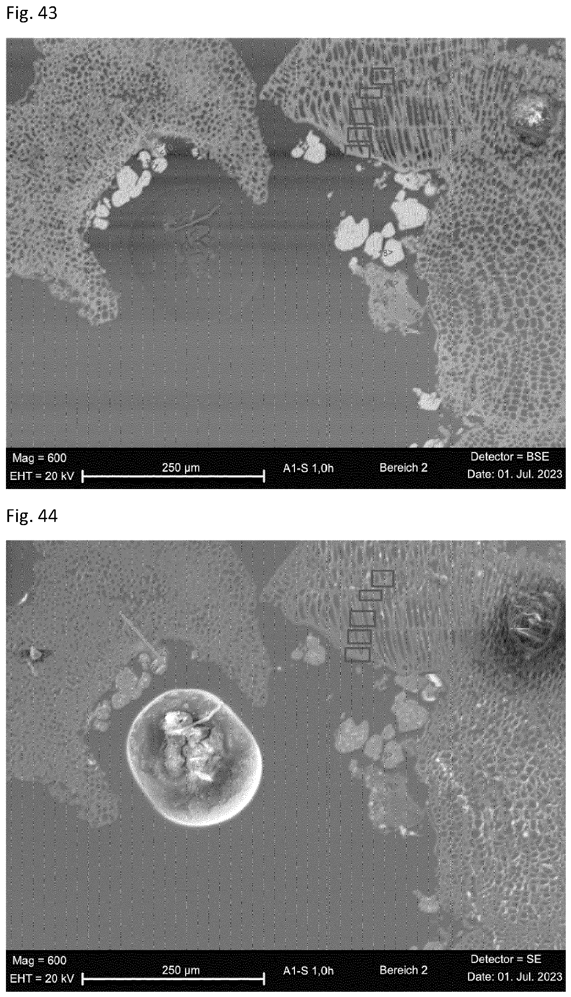

Abschließend betreffen die in den Figuren 33-44 gezeigten Algenmaterial, welches nach dem erfindungsgemäßen Verfahren für 1h / 900°C behandelt wurde. Hierbei zeigen die Figuren jeweils die analogen Analysen wie die zuvor beschriebenen Figuren.

Auch hier sind wieder die gleichen Phänomene zu sehen, wie sie oben bereits bei der Probe mit einer Behandlungsdauer von 0,5 Stunde erwähnt wurden.

Die Profile (Punkt 1 bis 4, 6 bis 8 und 19 bis 23) zeigen jeweils den Anstieg der Konzentrationen an Sr.

Beim Profil 19 bis 23 wurden keine Punkte, sondern kleine Flächen analysiert, die somit eine integrale Analyse der Fläche darstellen. Auch hier ist die Zunahme im Sr zu verzeichnen.

Bei beiden SrF₂-Körnern, die im Bild zu sehen sind, kann man erkennen, dass das Sr bis etwa 50 µm tief in das poröse Algenmaterial eindringt. Das ist deutlich mehr als bei dem kompakten Knochenmaterial.

Zusammenfassend kann festgehalten werden, dass in den vorliegenden Proben der Einbau von Sr in das Knochenmaterial und in das Algenmaterial bei den vorliegenden Versuchsbedingungen in sehr ausgeprägtem Maße erfolgt ist.

Man erkennt, dass das Sr manchmal recht tief in das Material eindringt und dabei das Ca aus dem Gitter verdrängt. Die stöchiometrische Berechnung der atomaren Anteile deutet an, dass hier ein einfacher Austausch der beiden Elemente gegeneinander erfolgt.

Die Bildung von sauerstoffreichen Säumen um die SrF₂-Körner lässt sich klar nachweisen. Dies geht hin bis zur vollständigen Verdrängung von SrF₂. Ob dieser Effekt eine Zwischenstufe zur Verdrängung des Ca aus dem Apatitgitter darstellt, kann nicht gesagt werden.

Figur 45 zeigt ein Diagramm, das den erfindungsgemäßen Temperatur- und Behandlungszeitbereich verdeutlicht. Erfindungsgemäß erfolgt die thermische Behandlung unter bestimmten Rahmenbedingungen. Diese sind durch einen Bereich definiert, der durch die Behandlungstemperatur und die Behandlungszeit festgelegt ist, wie in Figur 45 veranschaulicht. Die maximale Temperatur wird dabei durch den Schmelzpunkt der verwendeten Produkte bestimmt. So befindet sich der Temperatur- und Behandlungszeitbereich, in dem die erfindungsgemäße Reaktion erfolgreich abläuft rechts oberhalb der in der Figur gezeigten Kurve.

Gemäß der Erfindung kann das erfindungsgemäße Verfahren durchgeführt werden, wenn die Kombination aus Behandlungstemperatur und Behandlungszeit in dem Bereich liegt, dessen untere Grenze durch die Punkte 1150°C/2 min 900°C/3 min, 700°C/5 min, 600°C/7 min, 500°C/10 min und 400°C/30 min definiert ist. Mit anderen Worten kann die Erfindung z. B. bei 500 °C und 20 min ausgeführt werden, aber zumindest nicht mit den außergewöhnlich guten Ergebnissen, wie bei 550°C und 30min Behandlung. Demgegenüber kann die Erfindung bei einer Ausführung mit 400 °C und 20 min nicht erfolgreich durchgeführt werden.

## Patentansprüche

1. Verfahren zum Modifizieren eines Knochenersatzmaterials oder einer Oberflächenbeschichtung eines Implantats zu einem Produkt mit osteotropen Eigenschaften aus einem festen, mikroporösen Ausgangsmaterial, welches im Wesentlichen Hydroxylapatit (Ca₅[OH|(PO₄)₃] aufweist und in kristalliner Form makroskopisch sichtbar vorliegt, mit den in folgender Reihenfolge durchzuführenden Schritten:
• Zerkleinern eines Modifizierungsmaterials aus einer festen Quelle für Strontium- Fluor- Magnesium-, Mangan-, Zink und/oder Galliumatomen auf einen durchschnittlichen Durchmesser von maximal 1,0 mm oder kleiner,
• Aufbringen des zerkleinerten Modifizierungsmaterials auf das Ausgangsmaterial zum Herstellen eines Zwischenproduktes,
• thermisches Behandeln des Zwischenprodukts bei einer Behandlungstemperatur unterhalb des Schmelzpunktes des Modifizierungsmaterials und des Knochenersatzmaterials oder der Oberflächenbeschichtung des Implantats für eine Behandlungszeit in einer Einrichtung zum thermischen Behandeln in einer offenen Atmosphäre, so dass Feuchtigkeit entweichen kann, zum Herstellen des Produktes, wobei teilweise Calcium Atome des Hydroxylapatit durch Atome des Modifizierungsmaterials ersetzt werden,
∘ wobei das Modifizierungsmaterial des Zwischenproduktes eine maximale Feuchtigkeit von 10% aufweist und
∘ wobei die Behandlungstemperatur und die Behandlungszeit in einem Bereich liegen, dessen untere Grenze bei mindestens 400 °C Behandlungstemperatur und mindestens 30 min Behandlungszeit liegt und wobei der Bereich weiter durch die Kombinationen mindestens 500°C Behandlungstemperatur und mindestens 10 min Behandlungszeit, mindestens 600°C Behandlungstemperatur und mindestens 7 min Behandlungszeit, mindestens 700°C Behandlungstemperatur und mindestens 5 min Behandlungszeit, mindestens 900°C Behandlungstemperatur und mindestens 3 min Behandlungszeit sowie mindestens 1150°C Behandlungstemperatur und mindestens 2 min Behandlungszeit definiert ist,
• Reinigen des Produktes von nicht eingebauten Modifizierungsmaterial durch mechanische, chemische oder physikalische Reinigungsverfahren.

2. Verfahren nach Anspruch 1,
**dadurch gekennzeichnet,**
**dass** zum Aufbringen des zerkleinerten Modifizierungsmaterials auf das Ausgangsmaterial das zerkleinerte Modifizierungsmaterial zumindest teilweise in einer Flüssigkeit, bevorzugt Wasser, gelöst wird und auf das Ausgangsmaterial aufgebracht wird, wobei eine anschließende Trocknung zum Entfernen der Flüssigkeit durchgeführt wird.

3. Verfahren nach Anspruch 1,
**dadurch gekennzeichnet,**
**dass** zum Aufbringen des zerkleinerten Modifizierungsmaterials auf das Ausgangsmaterial das zerkleinerte Modifizierungsmaterial mit geringen Mengen einer Flüssigkeit versetzt wird, um eine schlammartige Konsistenz zu erzeugen und dieser Schlamm auf das Ausgangsmaterial aufgetragen wird.

4. Verfahren nach einem der Ansprüche 1 bis 3,
**dadurch gekennzeichnet,**
**dass** vor dem thermischen Behandeln des Zwischenproduktes überschüssiges zerkleinertes Modifizierungsmaterial mittels physikalischer Schritte entfernt wird.

5. Verfahren nach einem der Ansprüche 1 bis 4,
**dadurch gekennzeichnet,**
**dass** zum Aufbringen des zerkleinerten Modifizierungsmaterials auf das Ausgangsmaterial das Ausgangsmaterial im zerkleinerten Modifizierungsmaterial eingebettet wird und derart thermisch behandelt wird.

6. Verfahren nach einem der Ansprüche 1 bis 4,
**dadurch gekennzeichnet,**
**dass** zum Aufbringen des zerkleinerten Modifizierungsmaterials auf das Ausgangsmaterial trockenes zerkleinertes Modifizierungsmaterial aufgetragen wird.

7. Verfahren nach einem der Ansprüche 1 bis 6,
**dadurch gekennzeichnet,**
**dass** als Quellen für Strontium, Fluor, Magnesium, Mangan, Zink und/oder Galliumatome die jeweiligen Stoffe selbst oder chemische Verbindungen, vorzugsweise deren Salze, verwendet werden, die diese Atome aufweisen, insbesondere ausgewählt aus: Strontiumfluorid, Magnesiumfluorid, Zinkfluorid, Mangan, Galliumoxid.

8. Verfahren nach einem der Ansprüche 1 bis 7,
**dadurch gekennzeichnet,**
**dass** zum thermischen Behandeln des Zwischenproduktes Aufheiz- und/oder Abkühlintervalle vorgesehen sind, um die gewünschte Behandlungstemperatur beziehungsweise Raumtemperatur zu erreichen.

9. Verfahren nach einem der Ansprüche 1 bis 8,
**dadurch gekennzeichnet,**
**dass** die Aufheiz- und/oder Abkühltemperaturänderung maximal 10°C pro Minute, bevorzugt weniger als 5°C pro Minute, beträgt.

10. Verfahren nach einem der Ansprüche 1 bis 7,
**dadurch gekennzeichnet,**
**dass** zum thermischen Behandeln des Zwischenproduktes dieses direkt in eine Behandlungseinrichtung eingebracht wird, welche im Wesentlichen die Behandlungstemperatur aufweist.

11. Osteotropes Knochenersatzmaterial oder osteotrope Oberflächenbeschichtung eines Implantats hergestellt nach einem der Verfahren 1 bis 10,
**dadurch gekennzeichnet,**
**dass** Calziumatome des Hydroxylapatits durch Strontium-, Fluor-, Magnesium-, Mangan-, Zink- und/oder Galliumatome teilweise ersetzt sind.

## Claims

1. Method for modifying a bone replacement material or a surface coating of an implant into a product having osteotropic properties, from a solid, microporous starting material which substantially comprises hydroxyapatite (Ca₅[OH|(PO₄)₃] and is present in crystalline form so as to be macroscopically visible, with the steps to be carried out in the following order:
- comminuting a modification material from a solid source of strontium, fluorine, magnesium, manganese, zinc and/or gallium atoms to an average diameter of at most 1.0 mm or smaller,
- applying the comminuted modification material to the starting material in order to produce an intermediate product,
- thermally treating the intermediate product at a treatment temperature below the melting point of the modification material and of the bone replacement material or of the surface coating of the implant, for a treatment time, in a device for thermal treatment in an open atmosphere, so that moisture can escape, in order to produce the product, wherein calcium atoms of the hydroxyapatite are partially replaced by atoms of the modification material,
- wherein the modification material of the intermediate product exhibits a maximum moisture of 10% and
- wherein the treatment temperature and the treatment time lie within a range whose lower limit is at least 400 °C treatment temperature and at least 30 min treatment time, and wherein the range is further defined by the combinations of at least 500 °C treatment temperature and at least 10 min treatment time, at least 600 °C treatment temperature and at least 7 min treatment time, at least 700 °C treatment temperature and at least 5 min treatment time, at least 900 °C treatment temperature and at least 3 min treatment time, and at least 1150 °C treatment temperature and at least 2 min treatment time,
- cleaning the product of non-incorporated modification material by mechanical, chemical or physical cleaning methods.

2. Method according to claim 1,
**characterized in that**
for applying the comminuted modification material to the starting material, the comminuted modification material is at least partially dissolved in a liquid, preferably water, and is applied to the starting material, wherein a subsequent drying is carried out in order to remove the liquid.

3. Method according to claim 1,
**characterized in that**
for applying the comminuted modification material to the starting material, the comminuted modification material is admixed with small amounts of a liquid in order to produce a slurry-like consistency, and this slurry is deposited on the starting material.

4. Method according to any one of claims 1 to 3,
**characterized in that**
before the thermal treatment of the intermediate product, excess comminuted modification material is removed by means of physical steps.

5. Method according to any one of claims 1 to 4,
**characterized in that**
for applying the comminuted modification material to the starting material, the starting material is embedded in the comminuted modification material and is thermally treated in this manner.

6. Method according to any one of claims 1 to 4,
**characterized in that**
for applying the comminuted modification material to the starting material, dry comminuted modification material is deposited.

7. Method according to any one of claims 1 to 6,
**characterized in that**
the sources used for strontium, fluorine, magnesium, manganese, zinc and/or gallium atoms are the respective substances themselves or chemical compounds, preferably salts thereof, which comprise these atoms, in particular selected from: strontium fluoride, magnesium fluoride, zinc fluoride, manganese, gallium oxide.

8. Method according to any one of claims 1 to 7,
**characterized in that**
for thermally treating the intermediate product, heating and/or cooling intervals are provided in order to reach the desired treatment temperature or room temperature.

9. Method according to any one of claims 1 to 8,
**characterized in that**
the heating and/or cooling temperature change is at most 10 °C per minute, preferably less than 5 °C per minute.

10. Method according to any one of claims 1 to 7,
**characterized in that**
for thermally treating the intermediate product, the latter is introduced directly into a treatment device which substantially exhibits the treatment temperature.

11. Osteotropic bone replacement material or osteotropic surface coating of an implant produced by one of the methods 1 to 10,
**characterized in that**
calcium atoms of the hydroxyapatite are partially replaced by strontium, fluorine, magnesium, manganese, zinc and/or gallium atoms.

## Revendications

1. Procédé de modification d'une substance de substitution osseuse ou d'un revêtement de surface d'un implant en un produit présentant des propriétés ostéotropes, à partir d'une substance de départ solide et microporeuse qui comprend essentiellement de l'hydroxyapatite (Ca₅[OH|(PO₄)₃] et qui se présente sous forme cristalline de manière macroscopiquement visible, avec les étapes à exécuter dans l'ordre suivant :
- broyage d'une substance de modification provenant d'une source solide d'atomes de strontium, de fluor, de magnésium, de manganèse, de zinc et/ou de gallium jusqu'à un diamètre moyen de 1,0 mm au maximum ou inférieur,
- application de la substance de modification broyée sur la substance de départ afin de fabriquer un produit intermédiaire,
- traitement thermique du produit intermédiaire à une température de traitement inférieure au point de fusion de la substance de modification et de la substance de substitution osseuse ou du revêtement de surface de l'implant, pendant un temps de traitement, dans un dispositif de traitement thermique sous atmosphère ouverte, de sorte que l'humidité puisse s'échapper, afin de fabriquer le produit, dans lequel des atomes de calcium de l'hydroxyapatite sont partiellement remplacés par des atomes de la substance de modification,
- dans lequel la substance de modification du produit intermédiaire présente une humidité maximale de 10 % et
- dans lequel la température de traitement et le temps de traitement se situent dans une plage dont la limite inférieure est d'au moins 400 °C de température de traitement et d'au moins 30 min de temps de traitement, et dans lequel la plage est en outre définie par les combinaisons d'au moins 500 °C de température de traitement et d'au moins 10 min de temps de traitement, d'au moins 600 °C de température de traitement et d'au moins 7 min de temps de traitement, d'au moins 700 °C de température de traitement et d'au moins 5 min de temps de traitement, d'au moins 900 °C de température de traitement et d'au moins 3 min de temps de traitement ainsi que d'au moins 1150 °C de température de traitement et d'au moins 2 min de temps de traitement,
- nettoyage du produit de la substance de modification non incorporée par des procédés de nettoyage mécaniques, chimiques ou physiques.

2. Procédé selon la revendication 1,
**caractérisé en ce que**
pour appliquer la substance de modification broyée sur la substance de départ, la substance de modification broyée est au moins partiellement dissoute dans un liquide, de préférence de l'eau, et est appliquée sur la substance de départ, dans lequel un séchage ultérieur est effectué afin d'éliminer le liquide.

3. Procédé selon la revendication 1,
**caractérisé en ce que**
pour appliquer la substance de modification broyée sur la substance de départ, la substance de modification broyée est additionnée de faibles quantités d'un liquide afin de produire une consistance boueuse, et cette boue est déposée sur la substance de départ.

4. Procédé selon l'une quelconque des revendications 1 à 3,
**caractérisé en ce que**
avant le traitement thermique du produit intermédiaire, la substance de modification broyée en excès est éliminée au moyen d'étapes physiques.

5. Procédé selon l'une quelconque des revendications 1 à 4,
**caractérisé en ce que**
pour appliquer la substance de modification broyée sur la substance de départ, la substance de départ est noyée dans la substance de modification broyée et est traitée thermiquement de cette manière.

6. Procédé selon l'une quelconque des revendications 1 à 4,
**caractérisé en ce que**
pour appliquer la substance de modification broyée sur la substance de départ, de la substance de modification broyée sèche est déposée.

7. Procédé selon l'une quelconque des revendications 1 à 6,
**caractérisé en ce que**
les sources utilisées pour les atomes de strontium, de fluor, de magnésium, de manganèse, de zinc et/ou de gallium sont les substances elles-mêmes ou des composés chimiques, de préférence leurs sels, qui comprennent ces atomes, en particulier choisis parmi : fluorure de strontium, fluorure de magnésium, fluorure de zinc, manganèse, oxyde de gallium.

8. Procédé selon l'une quelconque des revendications 1 à 7,
**caractérisé en ce que**
pour le traitement thermique du produit intermédiaire, des intervalles de chauffage et/ou de refroidissement sont prévus afin d'atteindre la température de traitement souhaitée ou la température ambiante.

9. Procédé selon l'une quelconque des revendications 1 à 8,
**caractérisé en ce que**
la variation de température de chauffage et/ou de refroidissement est de 10 °C par minute au maximum, de préférence de moins de 5 °C par minute.

10. Procédé selon l'une quelconque des revendications 1 à 7,
**caractérisé en ce que**
pour le traitement thermique du produit intermédiaire, celui-ci est introduit directement dans un dispositif de traitement qui présente essentiellement la température de traitement.

11. Substance de substitution osseuse ostéotrope ou revêtement de surface ostéotrope d'un implant fabriqué selon l'un des procédés 1 à 10,
**caractérisé en ce que**
des atomes de calcium de l'hydroxyapatite sont partiellement remplacés par des atomes de strontium, de fluor, de magnésium, de manganèse, de zinc et/ou de gallium.
